# EUROPEAN PATENT SPECIFICATION

(11) **EP 0 975 227 B1**
(45) Date of publication and mention of the grant of the patent: **09.11.2005**
(21) Application number: 98918115.1
(22) Date of filing: 10.04.1998
(51) Int. Cl.: A61K 35/74, A61P 31/00, A61L 15/36

(54) **TOPICAL USE OF PROBIOTIC BACILLUS SPORES TO PREVENT OR CONTROL MICROBIAL INFECTIONS**
OBERFLÄCHIGE VERWENDUNG VON PROBIOTISCHEN BACILLUSSPOREN ZUR VERHINDERUNG ODER BEKÄMPFUNG VON MIKROBIELLEN INFEKTIONEN
UTILISATION TOPIQUE DE SPORES DE BACILLES PROBIOTIQUES POUR PREVENIR OU TRAITER LES INFECTIONS MICROBIENNES

(30) Priority: 18.04.1997 US 44643 P
(43) Date of publication of application: 02.02.2000
(73) Proprietor: Ganeden Biotech, Inc., La Jolla, CA 92037 (US)
(72) Inventor: FARMER, Sean, La Jolla, CA 92037 (US); MIKHAIL, Robert, J., El Cajon, CA 92019 (US)
(74) Representative: Crump, Julian Richard John
(86) International application number: PCT/US1998/007307
(87) International publication number: WO 1998/047374

(56) References cited:
- WO-A-93/14187
- WO-A-94/11492
- JP-A- 63 096 107
- US-A- 4 871 539
- US-A- 5 000 939
- US-A- 5 045 314
- US-A- 5 344 647
- US-A- 5 431 924
- US-A- 5 455 028
- DATABASE MEDLINE [Online] September 1995 (1995-09) MOLDENHAUER J E ET AL: "Heat resistance of Bacillus coagulans spores suspended in various parenteral solutions." Database accession no. NLM7489197 XP002225767 & PDA JOURNAL OF PHARMACEUTICAL SCIENCE AND TECHNOLOGY / PDA. UNITED STATES 1995 SEP-OCT, vol. 49, no. 5, September 1995 (1995-09), pages 235-238, ISSN: 1079-7440
- DATABASE WPI Section Ch, Week 199309 Derwent Publications Ltd., London, GB; Class B04, AN 1993-073946 XP002225769 & KR 9 205 685 B (ILDONG PHARM CO), 13 July 1992 (1992-07-13)
- DATABASE WPI Section Ch, Week 199734 Derwent Publications Ltd., London, GB; Class B04, AN 1997-367033 XP002225768 & JP 09 154535 A (SANWA KAGAKU KENKYUSHO CO LTD), 17 June 1997 (1997-06-17)
- DATABASE WPI Section Ch, Week 199637 Derwent Publications Ltd., London, GB; Class C05, AN 1996-368043 XP002133289 & JP 08 175921 A (IDEMITSU KOSAN CO LTD), 9 July 1996 (1996-07-09)
- SIEGEL et al., "Clearance of Bacillus Sphaericus and Bacillus Thuringiensis ssp. Israelensis from Mammals", JOURNAL OF ECONOMIC ENTOMOLOGY, April 1990, Vol. 83, No. 2, pages 347-355, XP002914649
- SYTNIK S.I. et al., "Antagonistic Action of Corinebacteria and Bacilli of Cutaneous Ecotype on Staphylococci", MIKROBIOLOGICHESKII ZHURNAL, January-February 1989, Vol. 51, No. 1, pages 82-87, XP002914650
- SCHOENI et al., "Inhibition of Campylobacter Jejuni Colonization in Chicks by Defined Competitive Exclusion Bacteria", APPLIED AND ENVIRONMENTAL MICROBIOLOGY, April 1994, Vol. 60, No. 4, pages 1191-1197, XP002914651

## Description

### Technical Field

This invention relates to utilizing a probiotic *Bacillus* coagulans organism in a therapeutic composition as a topical agent, and specifically relates to the use of compositions derived from *Bacillus coagulans* for prevention and control of microbial infections.

### Background of the Invention

Probiotic agents are organisms that confer a benefit when they grow in a particular environment, often by inhibiting the growth of other biological organisms in the same environment. Examples of probiotics include bacteria and bacteriophages which can grow in the intestine, at least temporarily, to displace or destroy pathogens and provide other benefits to the host organism (Salminen et al, Antonie Van Leeuwenhoek, 70 (2-4): 347-358, 1996; Elmer et al, JAMA, 275:870-876, 1996; Rafter, Scand. J. Gastroenterol., 30:497-502, 1995; Perdigon et al, J. Dairy Sci., 78:1597-1606, 1995; Gandi, Townsend Lett. Doctors & Patients, pp.108-110, Jan. 1994; Lidbeck et al, Eur. J. Cancer Prev. 1:341-353, 1992). Probiotic preparations were systematically evaluated for their effect on health and longevity in the early 1900's (Metchnikoff, E., *Prolongation of Life*, Willwn Heinemann, London, 1910; republished by G.P. Putnam's Sons, New York, NY, 1970). Since the discovery and widespread use of antibiotics in about 1950 to treat pathological microbes, the use of probiotics has been limited.

The widespread use of antimicrobial drugs, especially broad spectrum antibiotics, has produced serious consequences. Individuals taking antibiotics often suffer from gastrointestinal upset when beneficial microorganisms in the gut are killed, thus changing the balance of the intestinal flora. This imbalance can result in vitamin deficiencies when vitamin-producing gut bacteria are killed and/or illness when a pathogenic organism overgrows and replaces the beneficial gut microorganisms. In addition to gut microflora, beneficial and/or pathological microorganisms can inhabit the oral cavity, the genital area and the vagina (Thomason J.L. et al., *Am. J. Obstet. Gynecol.* 165 (4 Pt. 2):1210-1217, 1991; Marsh, P.D., *Caries Res*. 27 (Suppl. 1):72-76, 1993; Lehner T., *Vaccine* 3(1): 65-68, 1985; Hill L.V. & Embil, J.A., *Can. Med. Assoc. J.* 134(4):321-331, 1986). The use of antimicrobial drugs can similarly cause an imbalance in those microorganisms and the therapeutic use of probiotic bacteria, especially *Lactobacillus* strains, that colonize those areas has been disclosed (Winberg, J. et al., *Pediatr. Nephrol.* 7(5):509-514, 1993; Malin M. et al., *Ann. Nutr. Metab.* 40(3);137-145, 1996; U.S. Pat. No. 5,176,911).

Increasing numbers of pathogenic microorganisms have developed antibiotic resistance, requiring the development and use of second and third generation antibiotics. Microorganisms that are resistant to multiple drugs have also developed, often with multiple drug resistance spreading between species, leading to serious infections that cannot be controlled by use of antibiotics.

Opportunistic microbial infections often occur in inmunodeficient individuals. Immunodeficient individuals have impaired natural immunity allowing pathogenic microorganisms to survive and grow, either internally or externally, due to the individual's diminished immune response to the pathogen. Immunodeficiency can result from genetic conditions, diseases such as AIDS, or therapeutic treatments such as cancer therapy (chemotherapy or radiation treatment) and drug-mediated immunosuppression following organ transplant. Inhibition of pathogenic microorganisms by probiotics is useful for preventing or treating opportunistic infections, particularly in immunodeficient individuals.

Thus, there is a need for preventive and therapeutic agents that can control the growth of pathogenic microorganisms without the use of antibiotic chemicals to which the microorganisms already are or can become resistant. Probiotics can be applied either internally or externally to restore the balance of beneficial microorganisms to pathogens, without contributing to the evolution of drug-resistant pathogens.

Lactic acid producing bacteria (e.g., *Bacillus, Lactobacillus* and *Streptococcus* species) have been used as food additives and there have been some claims that they provide nutritional and therapeutic value (Gorbach S.L., *Ann. Med*. 22(1):37-41, 1990; Reid, G. et al., *Clin. Microbiol. Rev.* 3(4):335-344, 1990). Some lactic acid producing bacteria (e.g., those used to make yogurt) have been suggested to have antimutagenic and anticarcinogenic properties useful for preventing human tumors (Pool-Zobel B.L. et al., *Nutr. Cancer* 20(3):261-270, 1993; U.S. Pat. No. 4,347,240). Some lactic acid producing bacteria also produce bacteriocins which are inhibitory metabolites responsible for the bacteria's antimicrobial effects (Klaenhammer T.R., *FEMS Microbiol. Rev.* 12(1-3):39-85, 1993; Barefoot S.F. & Nettles C.G., *J. Dairy Sci.* 76(8):2366-2379, 1993).

Selected *Lactobacillus* strains that produce antibiotics have been disclosed as effective for treatment of infections, sinusitis, hemorrhoids, dental inflammations, and other inflammatory conditions (U.S. Pat. No. 4,314,995). *L reuteri* produces antibiotics with activity against Gram negative and Gram positive bacteria, yeast and a protozoan (U.S. Pat. No. 5,413,960 and U.S. Pat. No. 5,439,678). *L. casei* ssp. *rhamnosus* strain LC-705, DSM 7061, alone or in combination with a *Propionibacterium* species, in a fermentation broth has been shown to inhibit yeast and molds in food and silage (U.S. Pat. No. 5,378,458). Also, antifungal *Serratia* species have been added to animal forage and/or silage to preserve the animal feedstuffs, particularly *S. rubidaea* FB299, alone or combined with an antifungal *B. subtilis* (strain FB260) (U.S. Pat. No. 5,371,011).

*Bacillus coagulans* is a non-pathogenic gram positive spore-forming bacteria that produces L(+) lactic acid (dextrorotatory) in homofermentation conditions. It has been isolated from natural sources, such as heat-treated soil samples inoculated into nutrient medium (*Bergey's Manual of Systemic Bacteriology*, Vol. 2, Sneath, P.H.A. et al., eds., Williams & Wilkins, Baltimore, MD, 1986). Purified *B. coagulans* strains have served as a source of enzymes including endonucleases (e.g., U.S. Pat. No. 5,200,336), amylase (U.S. Pat. No. 4,980,180), lactase (U.S. Pat. No. 4,323,651) and cyclo-malto-dextrin glucano-transferase (U.S. Pat. No. 5,102,800). *B. coagulans* has been used to produce lactic acid (U.S. Pat. No. 5,079,164). A strain of *B. coagulans* (referred to as *L. sporogenes* Sakaguti & Nakayama (ATCC 31284)) has been combined with other lactic acid producing bacteria and *B. natto* to produce a fermented food product from steamed soybeans (U.S. Pat. No. 4,110,477). *B. coagulans* strains have also been used as animal feed additives for poultry and livestock to reduce disease and improve feed utilization and, therefore, to increase growth rate in the animals (International PCT Pat Applications No. WO 9314187 and No. WO 9411492).

### Summary of the Invention

It has now been discovered that *Bacillus* coagulans possess the ability to exhibit probiotic activity in aerobic conditions such as on skin or mucous membrane tissues and thereby treat, control and/or inhibit numerous conditions caused by microbial infections. The invention describes therapeutic compositions, articles of manufacture and methods of use for inhibiting various microbial infections caused by bacteria, yeast, fungus or virus, which utilize isolated *Bacillus* coagulans.

According to the invention, there is provided a composition comprising an isolated *Bacillus coagulans* bacterium in a pharmaceutically acceptable carrier suitable for topical application to skin or a mucous membrane of a mammal. In one embodiment of the composition, the *Bacillus coagulans* is included in the composition in the form of spores. In another embodiment, the *Bacillus coagulans* is included in the composition in the form of a dried cell mass. In the composition, the carrier may be an emulsion, cream, lotion, gel, oil, ointment, suspension, aerosol spray, powder, aerosol powder or semi-solid formulation.

According to a preferred aspect of the invention, there is provided a composition comprising an extracellular product of a *Bacillus coagulans* species in a pharmaceutically acceptable carrier suitable for topical application to skin or a mucous membrane of a mammal. In one embodiment, the extracellular product is a supernatant or filtrate of a culture of an isolated *Bacillus coagulans* species. The carrier may be an emulsion, cream, lotion, gel, oil, ointment, suspension, aerosol spray, powder, aerosol powder or semi-solid formulation.

According to another aspect of the invention, there is provided use of a composition comprising a *Bacillus coagulans* bacterium or extracellular product thereof in a pharmaceutically acceptable carrier for the manufacture of a medicament for preventing bacterial, yeast, fungal or viral infection, wherein the medicament is suitable for topical application to skin or a mucous membrane of a mammal, and wherein the carrier is in the form of an emulsion, cream, lotion, gel, oil, ointment, suspension, aerosol spray, powder, aerosol powder or semi-solid formulation.

In a further embodiment the *Bacillus coagulans* bacterium is included in the composition in the form of spores. In one embodiment, the medicament inhibits growth of one or more microbe species selected from the group consisting of *Staphylococcus* species, *Streptococcus* species, *Pseudomonas* species, *Escherichia coli, Gardnerella vaginalis, Propionibacterium acnes, Aeromonas hydrophilia, Aspergillus* species, *Proteus* species, *Aeromonas* species, *Clostridium* species, *Klebsiella* species, *Candida* species and *Trichophyton* species. Also inhibited are certain virus species.

According to another aspect of the invention, there is provided use of a composition comprising an extracellular product of an isolated *Bacillus coagulans* species in a pharmaceutically acceptable carrier for the manufacture of a medicament for inhibiting growth of bacteria, yeast, fungus, virus or a combination thereof, wherein the medicament is suitable for topical application to skin or a mucous membrane of a mammal, and wherein the carrier is in the form of an emulsion, cream, lotion, gel, oil, ointment, suspension, aerosol spray, powder, aerosol powder or semi-solid formulation.

According to another aspect of the invention, there is provided a composition comprising an isolated *Bacillus coagulans* bacterium or extracellular product thereof applied to a flexible article that is intended to be worn by or attached to skin or a mucous membrane of a mammal to allow probiotic activity of the isolated *Bacillus coagulans* bacterium or extracellular product thereof to occur adjacent to or on the skin or mucous membrane.

According to another aspect of the invention, there is provided use of a composition comprising an isolated *Bacillus coagulans* bacterium or extracellular product thereof applied to a solid surface for the manufacture of a medicament for inhibiting growth of bacteria, yeast, fungus, virus or a combination thereof wherein the medicament is suitable for application to skin or mucous membrane of a mammal. In one embodiment, the application to a solid surface includes applying the composition to a diaper, pliable material for wiping skin or a mucous membrane, dermal patch, adhesive tape, absorbent pad, tampon or article of clothing. In another embodiment, the application to a solid surface includes impregnating the composition into a fibrous or nonfibrous solid matrix.

The invention provides several advantages. In particular, insofar as there is a detrimental effect to the use of antibiotics because of the potential to produce antibioticresistant microbial species, it is desirable to have an antimicrobial therapy which does not utilize conventional antimicrobial reagents. The present invention does not contribute to the production of future generation of antibiotic resistant pathogens.

It should be understood that both the foregoing general description and the following detailed description are exemplary and explanatory only and are not restrictive of the invention as claimed.

### Detailed Description of the Invention

The present invention is directed to the discovery that *Bacillus* coagulans can be used in therapeutic compositions as a probiotic for preventing or controlling microbial infections. As discussed further, the compositions can be formulated in many configurations because the bacterium is presented as a viable organism, either as a vegetative cell or as a spore, and colonizes the tissue of interest. The cells/spores can be presented in compositions suited for topical application to a tissue, or in suspensions such as a bath, or on flexible materials such as diapers, bandaids, tampons and the like personal articles, all directed at the objective of introducing the bacteria topically to skin or a mucous membrane tissue.

*A Bacillus* species is particularly suited for the present invention due to the properties in common between species of the *Bacillus* genus, including in particular the ability to form spores which are relatively resistant to heat and other conditions, making them ideal for storage (shelf-life) in product formulations, and ideal for survival and colonization of tissues under conditions of pH, salinity, and the like on tissues subjected to microbial infection. Additional useful properties include non-pathogenic, aerobic, facultative and heterotrophic, rendering these species safe, and able to colonize skin and mucous membrane tissues.

There are a variety of different *Bacillus* coagulans species, including, but not limited to many different strains available through commercial and public sources, such as the American Tissue Culture Collection (ATCC). For example, *Bacillus coagulans* strains are available as ATCC Accession Numbers 15949, 8038, 35670, 11369, 23498, 51232, 11014, 31284, 12245, 10545 and 7050.

The growth of these various *Bacillus* species to form cell cultures, cell pastes and spore preparations is generally well known in the art. Exemplary culture and preparative methods are described herein for *Bacillus coagulans* and can readily be used for the other *Bacillus* species.

Exemplary methods and compositions are described herein using *Bacillus coagulans* as a probiotic for controlling, treating or reducing microbial infections.

As used herein, "probiotic" refers to microorganisms (e.g., bacteria, yeast, viruses and/or fungi) that form at least a part of the transient or endogenous flora and, thus, have a beneficial prophylactic and/or therapeutic effect on the host organism. Probiotics are generally known to be safe by those skilled in the art. Although not wishing to be bound by any particular mechanism, the probiotic activity of *Bacillus* species is thought to result from competitive inhibition of growth of pathogens due to superior colonization, parasitism of undesirable microorganisms, lactic acid production and/or other extracellular products having antimicrobial activity, or combinations thereof. These products and activities *of Bacillus* may act synergistically to produce the beneficial probiotic effect.

### A. Bacillus coagulans Compositions

We have demonstrated that purified *Bacillus cargulans* is exemplary and preferred as a probiotic for biological control of various microbial pathogens.

Because *B. coagulans* forms heat-resistant spores, it is particularly useful for making pharmaceutical compositions for treating microbial infections. Topical formulations that include viable *B. coagulans* spores in a pharmaceutically acceptable carrier are particularly preferred for making and using both preventive and therapeutic compositions. The term "topical" is used broadly to include both epidermal and/or skin surfaces, as well as mucosal surfaces of the body.

*B. coagulans* is non-pathogenic and is generally regarded as safe (i.e., GRAS classification by the U.S. Food and Drug Administration). The Gram positive rods have a cell diameter of greater than 1.0 µm with variable swelling of the sporangium, without parasporal crystal production.

### 1. Growth of B. coagulans

*B. coagulans* is aerobic and facultative, grown typically in nutrient broth, pH 5.7 to 6.8, containing up to 2% (by wt) NaCl, although neither NaCl nor KCl are required for growth. A pH of about 4 to about 6 is optimum for initiation of growth from spores. It is optimally grown at about 30°C to about 55°C, and the spores can withstand pasteurization. It exhibits facultative and heterotrophic growth by utilizing a nitrate or sulphate source. Additional metabolic characteristics of *B. coagulans* are summarized in Table 1.

**TABLE 1**

| Charactewristic | *B. coagulans* Response |
|---|---|
| Catalase production | Yes |
| Acid from D-Glucose | Yes |
| Acid from L-Arabinose | Variable |
| Acid from D-Xylose | Variable |
| Acid from D-Mannitol | Variable |
| Gas from Glucose | Yes |
| Hydrolysis of Casein | Variable |
| Hydrolysis of Gelatin | No |
| Hydrolysis of Starch | Yes |
| Utilization of Citrate | Variable |
| Utilization of Propionate | No |
| Degradation of Tyrosine | No |
| Degradation of Phenylalanine | No |
| Nitrate reduced to Nitrite | Variable |
| Allatoin or Urate Required | No |

*B. coagulans* can be grown in a variety of media, although it has been found that certain growth conditions produce a culture which yields a high level of sporulation. For example, sporulation is enhanced if the culture medium includes 10 milligrams per liter of manganese sulfate, yielding a ratio of spores to vegetative cells of about 80:20. In addition, certain growth conditions produce a bacterial spore which contains a spectrum of metabolic enzymes particularly suited for the present invention, i.e., control of microbial infections. Although spores produced by these particular growth conditions are preferred, spores produced by any compatible growth conditions are suitable for producing a *B. coagulans* useful in the present invention.

Suitable media for growth of *B. coagulans* include Nutristart 701, PDB (potato dextrose broth), TSB (tryptic soy broth) and NB (nutrient broth), all well known and available from a variety of sources. Media supplements containing enzymatic digests of poultry and fish tissue, and containing food yeast are particularly preferred. A preferred supplement produces a media containing at least 60% protein, and about 20% complex carbohydrates and 6% lipids. Media can be obtained from a variety of commercial sources, notably DIFCO (Detroit, MI), Oxoid (Newark, NJ), BBL (Cockeyesville, MD) and Troy Biologicals (Troy, MI).

A preferred procedure for preparation *of B. coagulans* is as follows. *B. coagulans* Hammer bacterium was inoculated and grown in nutrient broth containing 5 g Peptone, 3 g Meat extract, 10-30 mg MnSO₄ and 1,000 ml distilled water, adjusted to pH 7.0, using a standard airlift fermentation vessel at 30°C. The range of MnSO₄ acceptable for sporulation is 1 mg/l to 1 g/l. The vegetative cells can actively reproduce up to 65°C, and the spores are stable up to 90°C. After fermentation, the *B. coagulans* Hammer bacterial cells are collected using standard methods (e.g., filtration, centrifugation) and the collected cells and spores can be lyophilized, spray dried, air dried or frozen. As described herein, the supernatant from the cell culture can be collected and used as an extracellular agent secreted by *B. coagulans* which has antimicrobial activity useful in a formulation of this invention.

A typical yield from the above culture is about 100 to 150 billion cells/spores per gram before drying. Spores maintain at least 90% viability after drying when stored at room temperature for up to seven years, and thus the effective shelf life of a composition containing *B. coagulans* Hammer spores at room temperature is about 10 years.

### 2. Extracellular Products Having Antimicrobial Activity

*B. coagulans* cultures contain secreted products which have antimicrobial activity. These secreted products are useful in therapeutic compositions according to the present invention. Cell cultures are harvested as described above, and the culture supernatants are collected, by filtration or centrifugation, or both, and the resulting supernatant contains antimicrobial activity useful in a therapeutic composition. The preparation of a *B. coagulans* extracellular product is described in the Examples.

### 3. Sources of B. coagulans

Purified *B. coagulans* bacterium are available from the American Type Culture Collection (Rockville, MD) using the following accession numbers: *B. cargulans* Hammer NRS T27 (ATCC# 11014), *B. coagulans* Hammer strain C (ATCC# 11369), *B. coagulans* Hammer (ATCC# 31284), and *B. coagulans* Hammer NCA 4259 (ATCC# 15949). Purified *B. cargulans* bacterium are also available from the Deutsche Sammlung von Mikroorganismen und Zellkuturen GmbH (Braunschweig, Germany) using the following accession numbers: *B. coagulans* Hammer 1915^{AL} (DSM# 2356), *B. cargulans* Hammer 1915^{AL} (DSM# 2383, corresponds to ATCC# 11014), *B. coagulans* Hammer^{AL} (DSM# 2384, corresponds to ATCC# 11369), and *B. coagulans* Hammer^{AL} (DSM# 2385, corresponds to ATCC# 15949). *B. coagulans* bacterium can also be obtained from commercial suppliers such as Sabinsa Corporation (Piscataway, NJ).

These *B. coagulans* strains and their growth requirements have been described previously (Baker et al, Can. J. Microbiol. 6:557-563, 1960; Blumenstock, "*Bacillus* *coogulans* Hammer 1915 und andere thermophile oder mesophile, säuretolerante *Bacillus*-Arten-eine taxonomische Untersuchung", Doctoral thesis, Univ. Göttingen, 1984; Nakamura et al, Int. J. Syst. Bacteriol., 38:63-73, 1988). Strains of *B. coagulans* can also be isolated from natural sources (e.g., heat-treated soil samples) using well known procedures (*Bergey's Manual of Systemic Bacteriology*, Vol. 2, p. 1117, Sneath, P.H.A. et al., eds., Williams & Wilkins, Baltimore, MD, 1986). The results described herein were obtained with *B. coagulans* Hammer obtained from the American Type Culture Collection (ATCC# 31284) which was grown as described herein and stored in lyophilized aliquots at -20°C. All *B. coagulans* that exhibit the properties described herein are considered equivalents of this strain.

*B. coagulans* had previously been mischaracterized as a Lactobacillus in view of the fact that as originally described, this bacterium was labeled as *Lactobacillus sporogenes* (See Nakamura et al, cited above). However, this was incorrect because the bacterium of this invention produces spores and through metabolism excretes L(+)-lactic acid, both aspects which provide key features to its utility. Instead, these developmental and metabolic aspects required that the bacterium be classified as a lactic acid bacillus, and therefore it was renamed.

### 4. Probiotic Antimicrobial Activity of B. coagulans

Pathogenic bacteria inhibited by *B. coagulans* activity include *Staphylococcus aureus, S. epidermidis, Streptococcus pyogenes, S. spp., Pseudomonas aeruginosa, Escherichia coli* (enterohemorragic species), *Clostridium perfingens, C. difficile, Gardnerella vaginalis, Propionibacterium acnes, Aeromonas hydrophilia, Aspergillus* species, *Proteus* species and *Klebsiella* species. Pathogenic yeast and other fungus inhibited by *B. coagulans* activity include *Candida albicans, C. tropicalis* and *Trichophyton mentogrophytes, T. interdigitale, T. rubrum,* and *T. yaoundei. B. coagulans* activity also inhibits Herpes simplex viruses I and II. These pathogens can cause diaper rash, oral, genital, cervical and vaginal yeast infections, toxic shock syndrome, chronic mucocutaneous candidiasis, dermatophytosis, bacterial vaginosis, tineal fungal infections such as ringworm, athlete's foot and jock itch, scalp and nail fungal infections, superficial skin disorders such as erysipelas, open wound infections, acne, abscess, boil, eczema, dermatitis, contact dermatitis, hypersensitinitis, contact lesions, bed sores, diabetic lesions, miscellaneous opportunistic infections, oral and genital viral lesions, and the like conditions as are well known in the art. Therefore, topical use of compositions containing the *B. coagulans* active agents that inhibit these pathogens are useful in preventing or treating these conditions.

Antimicrobial activity of a therapeutic composition of this invention against many of the above-described pathogens is described in the Examples. In addition, it is contemplated that the present therapeutic compositions can be used, when formulated for administration to the relevant tissue, to treat infections as described below:

| Infecting Microbe | Condition |
|---|---|
| *Trichophyton* species | |
| *T. mentagrophytes* | tinea pedis, athlete's foot |
| *T. interdigitale* | tinea pedis, athlete's foot |
| *T. mentagrophytes* | tinea versicolor, ring worm |
| *T. mentagrophytes* | tinea barbae, face/neck inflammation |
| *T. rubrum* | dermatophytosis |
| *T. yaoundei* | Ring worm on scalp |
| | |

| *Candida* species | |
|---|---|
| *C. albicans* | systemic candidiasis |
| *C. albicans* | chronic mucocutaneous candidiasis, myositis and thymoma |
| *C. albicans* | yeast and mycelial phase infection |
| *C. albicans* | oral thrush |
| *C. tropicalis* | cervical yeast infection |
| | |
| *Pseudomonas aeruginosa* | opportunistic skin infections, urinary tract infections, post surgical infections |
| *Staphylococcus aureus* | opportunistic skin infections, abscess, boils, wound infections, dermatitis |
| *Staphylococcus epidermidis* | opportunistic skin infections |
| | |
| *Streptococcus pyogenes* | opportunistic skin infections, impetigo, erysipelas |
| *Streptococcus spp.* | opportunistic skin infections, wound infections |
| | |
| *Gardnerella vaginalis* | bacterial vaginosis |
| | |
| *Propionibacterium acnes* | acne |
| | |
| *Clostridium perfringens* | open wound infections |
| | |
| Herpes Simplex Virus I or II | cold sores, genital herpes lesions |

Other skin and mucous membrane infecting microbes and dermatophytes can also be treated using the present compositions and methods.

### B. Fructooligosaccharides

Fructooligosaccharides (F'S) are a class of sugars particularly useful in the context of the present invention. F'S are a simple class of natural carbohydrates comprising polymers of fructose and glucose. FOS are non-digestible, fructose polymers that are utilized almost exclusively by the indigenous Bifidobacteria and Lactobacillus in the intestinal tract and can be similarly utilized by *Bacillus.* Deleterious bacteria such as Clostridium, Staphylococcus, Salmonella and E.Coli cannot metabolize FOS and therefor use of FOS in combination with *Bacillus* allows the beneficial and probiotic bacteria to grow and to replace any undesirable or pathogenic microorganisms.

The use of FOS in therapeutic compositions of the present invention provides a synergistic effect thereby increasing the effectiveness of the *Bacillus*-containing compositions of this invention. This synergy is manifest at least by increasing the ability of the bacterium to grow by increasing the food supplement for *Bacillus* which preferentially selects for growth of *Bacillus* over many other bacteria in the infected tissue. Thus, the presence of FOS in the formulation allows for more effective microbial inhibition by increasing the ability of *Bucillus* to grow and therefore provide its benefit.

FOS can be obtained from a variety of natural sources, including commercial suppliers. As a product isolated from natural sources, the components can vary widely and still provide the beneficial agent, namely FOS. FOS typically has a polymer chain length of from about 4 to 200 sugar units, with the longer lengths being preferred. For example, the degree of purity can vary widely so long as functional FOS is present in the formulation. Preferred FOS formulations contain at least 50 % by weight of fructooligosaccharides compared to simple(mono or disaccharide) sugars such as glucose, fructose or sucrose, preferably at least 80 % fructooligosaccharides, more preferably at least 90 % and most preferably at least 95 % fructooligosaccharides. Sugar content and composition can be determined by any of a variety of complex carbohydrate analytical detection methods as is well known.

Preferred sources of FOS include inulin, Frutafit IQ (tm) from Imperial Suiker Unie (Sugar Land, Texas), NutraFlora (tm) from Americal Ingredients, Inc., (Anaheim, CA), Fabrchem, Inc., (Fairfield, CT), and Fruittrimfat Replacers and Sweeteners (Emeryville, CA).

### C. Therapeutic Compositions

Compositions of this invention suitable for use in preventing, treating or contolling microbial infections comprise an active ingredient that is *Bacillus coagulans*, *Bacillus coagulans spores*, extracellular antimicrobial or antibiotic metabolites of *B. coagulans*, or combinations thereof in various formulations.

The active *Bacillus* ingredients comprise about 0.1% to about 50% by weight of the final composition, preferably 1% to 10% by weight, in a formulation suitable for topical administration.

The formulation for a therapeutic composition of this invention may include other probiotic agents or nutrients for promoting spore germination and/or *Bacillus* growth. The compositions may also include known antimicrobial agents, known antiviral agents, known antifungal agents, all of which must be compatible with maintaining viability of the *Bacillus* active agent when *Bacillus* organisms or spores are the active agent. The other agents in the compositions can be either synergists or active agents. Preferably, the known antimicrobial, antiviral and/or antifungal agents are probiotic agents compatible with *Bacillus.* The compositions may also include known antioxidants, buffering agents, sunscreens and cosmetic agents, including coloring agents, fragrances, oils, essential oils, lubricants, moisterizers or drying agents. Antioxidants such as vitamin E may be included. Sunscreens such as para-aminobenzoic acid may be included. Lubricants such as synthetic or natural beeswax may also be included. Thickening agents may be added to the compositions such as polyvinylpyrrolidone, polyethylene glycol or carboxymethylcellulose.

Fragrances and essential oils are particulalry suited for the compositions used in personal hygiene products and methods, and can include sea salts, herbs or herb extracts, fragrance oils from a large variety of plants or animals, and fragrances from a large variety of plants or animals, as are all well known.

Preferred fragrances useful in a composition of this invention include african violet, frankincense & myrrh, lavender, vanilla, gardenia, honeysuckle, sandlewood, musk, jasmine, lotus, orange blossom, patchouli, heather, magnolia, amber, rose, and the like fragrances.

Preferred oils, including essential or fragrant oils, include almond, aloe, amber, apple, apricot, bayberry, benzion, cactus blossom, carnation, carrageenan, cedarwood, cinammon, cloves, coconut, cedar, copal, emu, eucalyptus, franfipani, frankincense & myrrh, gardenia, grapefruit, heather, herbs, honeysuckle, jasmine, jojoba, kelp, lavender, lemon, lilac, lotus, magnolia, mulberry, musk, myrrh, narcissus, orange blossom, patchouli, peach, pinon pine, plumeria, rose, rosemary, safflower, sage, sandalwood, spirulina, strawberry, vanilla, violet, wisteria, and the like oils. A particularly preferred oil for use in a composition of the invention is emu oil, typically used in an amount of about 1% to 75% by weight.

In addition, the fragrances and essential oils can be provided in various bath salt and bath soap compositions. Salts and soaps are also well known in the art and can include sea salts, desert salts, mineral salts, sodium sesquicarbonate, magnesium sulfate, and the like commonly used bath salts.

Fragrances, oils and salts are well known in the art, can be obtained from a variety of natural and commercial sources, and are not considered to limiting to the invention. Exemplary commercial sources include Innovative Body Science (Carlsbad, CA), Scents of Paradise SunBurst Technology, Inc., (Salem, OR), Intercontinental Fragrances, Inc., (Houston, TX), Scentastics, Inc., (Ft. Lauderdale, FL), Michael Giordano International, Inc., (North Miami, FL).

Chemicals used in the present compositions can be obtained from a variety of commercial sources, including Spectrum Quality Products, Inc (Gardena, CA), Seltzer Chemicals, Inc., (Carlsbad, CA) and Jarchem Industries, Inc., (Newark, NJ).

The active agents are combined with a carrier that is physiologically compatible with the skin, membrane or mucosal tissue of a human or animal to which it is topically administered. That is, the carrier is preferably substantially inactive except for surfactant properties used in making a suspension of the active ingredients. The compositions may include other physiologically active constituents that do not interfere with the efficacy of the active agents in the composition.

A typical therapeutic compostion will contain in a one gram dosage formulation from 10³ to 10¹², preferably 2 x 10⁵ to 10¹⁰, colony forming units (CFU) of viable *Bacillus* bacteria (i.e., vegetative cell) or bacterial spore. In one preferred embodiment a therapeutic composition may include from about 10 milligrams (mg) to one gram of fiuctooligosaccharides. The formulation may be completed in weight using any of a variety of carriers and/or binders. A preferred carrier is micro-crystalline cellose (MCC) added in an amount sufficient to complete the one gram dosage total weight. Particularly preferred formulations for a therapeutic composition of this invention are described in the Examples.

Carriers can be solid-based dry materials for formulations in powdered form, and can be liquid or gel-based materials for formulations in liquid or gel forms, which forms depend, in part, upon the routes or modes of administration.

Typical carriers for dry formulations include trehalose, malto-dextrin, rice flour, micro-crystalline cellulose (MCC), magnesium sterate, inositol, FOS, gluco-oligosaccharides (GOS), dextrose, sucrose, talc, and the like carriers.

Where the composition is dry and includes evaporated oils that produce a tendency for the composition to cake (adherence of the component spores, salts, powders and oils), it is preferred to include dry fillers which distribute the components and prevent caking. Exemplary anti-caking agents include MCC, talc, diatomaceous earth, amorphous silica and the like, typically added in an amout of from about 1 to 95 % by weight.

Suitable liquid or gel-based carriers are well known in the art, such as water and physiological salt solutions, urea, alcohols and glycols such as methanol, ethanol, propanol, butanol, ethylene glycol and propylene glycol, and the like. Preferably, water-based carriers are about neutral pH.

Suitable carriers include aqueous and oleaginous carries such as, for example, white petrolatum, isopropyl myristate, lanolin or lanolin alcohols, mineral oil, fragrant or exxential oil, nasturtium extract oil, sorbitan mono-oleate, propylene glycol, cetylstearyl alcohol (together or in various combinations), hydroxypropyl cellulose (MW = 100,000 to 1,000,000), detergents (e.g., polyoxyl stearate or sodium lauryl sulfate) and mixed with water to form a lotion, gel, cream or semi-solid composition. Other suitable carriers comprise water-in-oil or oil-in-water emulsions and mixtures of emulsifiers and emollients with solvents such as sucrose stearate, sucrose cocoate, sucrose distearate, mineral oil, propylene glycol, 2-ethyl-1,3-hexanediol, polyoxypropylene-15-stearyl ether and water. For example, emulsions containing water, glycerol stearate, glycerin, mineral oil, synthetic spermaceti, cetyl alcohol, butylparaben, propylparaben and methylparaben are commercially available. Preservatives may also be included in the carrier including methylparaben, propylparaben, benzyl alcohol and ethylene diamine tetraacetate salts. Well-known flavorings and/or colorants may also be included in the carrier. The composition may also include a plasticizer such as glycerol or polyethylene glycol (MW=800 to 20,000). The composition of the carrier can be varied so long as it does not interfere significantly with the pharmacological activity of the active ingredients or the viability of the *Bacillus* cells or spores.

A therapeutic composition can be formulated to be suitable for application in a variety of ways, for example in a cream for skin (e.g., ringworm or athlete's foot), in a wash for the mouth (e.g., oral thrush), in a douche for vaginal application (e.g., vaginitis), in a powder for chaffing (e.g., dermatitis), in a liquid for toe nails (e.g., tinea pedis), in a bath salt or bath powder for treating genital, foot or other tissue infections in a bath, and the like as described in more detail in the Examples. Other formulations will be readily apparent to one skilled in the art.

### D. Therapeutic Methods for Treating Microbial Infections

The present invention contemplates a method for treating, reducing or controlling microbial infections in a variety of skin and mucosal membrane tissues using a therapeutic composition or therapeutic article of manufacture of this invention. Optimally the compositions effectively reduce the yeast, fungal and/or viral titre in the treated individual, particularly at the site of application of the topical composition. For example, the pathogenic microbial titre in lesions is significantly reduced with topical treatment of affected areas of the skin or mucous membrane. The disclosed methods of treatment also reduce symptoms of pathogenic microbial infection (e.g., pain associated with infected or microbial-caused lesions) and promote more rapid healing than seen without *Bacillus* treatment.

The method of the present invention includes administration of a composition containing the active *Bacillus* ingredient to a human or animal to treat or prevent microbial, i.e, bacterial, yeast, fungal or viral, infection. Administration is preferably to the skin or a mucous membrane using a cream, lotion, gel, oil, ointment, suspension, aerosol spray, powder, semi-solid formulation (e.g., a suppository), or article of manufacture, all formulated to contain a therapeutic composition of this invention using methods well known in the art.

Application of the compositions containing the active *Bacillus* agent effective in preventing or treating a microbial infection generally consist of one to ten applications of 10 mg to 10 g of a composition per application for one day up to one month. Applications are generally once every twelve hours and up to once every four hours. Preferably two to four applications of the composition per day, of about 0.1 g to 5 g per application, for one to seven days are sufficient to prevent or treat a microbial infection. For topical applications, the compositions are preferably applied to lesions daily as soon as symptoms (e.g., pain, swelling or inflammation) are detected. Of course, the specific route, dosage and timing of the application will depend, in part, on the particular pathogen and/or condition being treated and the extent of the condition.

A preferred method involves the application of from 10³ to 10¹² viable bacterium or spore per day, preferably from 10⁵ to 10¹⁰, and more preferably about from 5 x 10⁸ to 10⁹ viable bacterium or spore per day. In addition, a preferred method optionally comprises application of a composition that aditionally contains from 10 mgs to 20 gms of fructooligosaccharide per day, preferably about 50 mg - 10 gm, and more preferably about from 150 mgs to 5 gms of fructooligosaccharide per day, to promote growth of the probiotic *Bacillus* species over the growth of the pathogen..

In the case of a therapeutic bath, one embodiment provides for the addition and admixing of a composition of dry *Bacillus* spores to a prepared bath that may contain soaps, oils, fragrances, salts, and the like bath components, followed by contacting the infected tissue to the bath water, as by "taking a bath" in the conventional sense. In this embodiment, the therapeutic probiotic spores can be packaged in a system with instructions as described herein. A typical bath would provide 10⁸ to 10¹⁰ CFU of bactial cells or spores, preferably about 1x10⁹ to 5x10⁹ CFU of cells or spores per bath.

Specific methods for treating a microbial infection are described in the Examples, and include diaper rash, vaginal yeast infection, opportunistic skin infection, tineal fungal infection, superficial skin infection, acne, cold sores, genital Herpes lesions, athlete's foot, and the like.

Unless defined otherwise, all scientific and technical terms used herein have the same meaning as commonly understood by those skilled in the relevant art. Unless mentioned otherwise, the techniques employed or contemplated herein are standard methodologies well known to one of ordinary skill in the art. The examples of embodiments are for illustration only.

### E. Therapeutic Systems for Treating Microbial Infections (Not part of the claimed invention)

The invention further contemplates a therapeutic system for treating, reducing and/or controlling microbial infections comprising a container comprising a label and a therapeutic composition according to the present invention, wherein said label comprises instructions for use of the composition for treating said infection.

Typically, the system is present in the form of a package containing a therapeutic composition of this invention, or in combination with packaging material. The packaging material includes a label or instructions for use of the components of the package. The instructions indicate the contemplated use of the packaged component as described herein for the methods or compositions of the invention.

For example, a system can comprise one or more unit dosages of a therapeutic composition according to the invention. Alternatively, the system can contain bulk quantities of a therapeutic composition. The label contains intructions for using the thereapeutic composition in either unit dose or in bulk forms as appropriate, and may include information regarding storage of the composition, disease indications, dosages, routes and modes of administration and the like information.

Furthermore, depending upon the particular contemplated use, the system may optionally contain either combined or in separate packages one or more of the following components: FOS: bath salts, soaps and oils (for a bath use), and the like components. One particularly preferred system comprises unit dose packages of *Bacillus* spores for use in combination with a conventional bath salt or bath soap product, together with instructions for using the *Bacillus* probiotic in a therapeutic method.

### F. Articles of Manufacture

The invention also contemplates various articles of manufacture which utilize the beneficial aspects of the present invention by combination of the therapeutic composition with various medical or personal hygiene devices so as to reduce or prevent microbial infections associated with the use of these devices. The invention comprises compositions of *B. coagulans* active agent applied to a solid surface or impregnated into a solid matrix of any device or article of manufacture that is intended to be in contact with skin or a mucous membrane. Preferably the solid surface is a flexible article than can be worn on or wiped on the skin or mucous membrane. More preferably, when the flexible item carrying the *Bacillus* and/or the isolated active agent is to be worn on the skin it includes a means for attaching the article to the skin such as, for example, an adhesive layer, interengaging hook and pile (Velcro®) connectors, or other well known means of attachment such as ties, snap closures, elastic, buttons and the like.

Specific embodiments which include *B. coagulans* active agent are diapers, towelettes (e.g., baby wipes or feminine hygiene towelettes), tampons, dermal patches, adhesive tape, absorbent pads, articles of clothing (e.g., underclothes, sleeping apparel), bath towels, wash cloths, and the like. The article may be made of fibrous woven, knitted or nonwoven materials, occlusive or nonocclusive films or membranes, synthetic polymer fibers, films, membranes and foams (e.g., nylon, polytetrafluoroethylene (PTFE, such as Teflon® or Gor-Tex®), polystyrene, polycarbonate, polyvinylchloride and polysulphone). All of these forms are well known in the art and include, for example, knitted or woven fabrics, nonwoven fabrics such as felt and batting, fiber balls of cotton, rayon, cellulose or synthetic fibers and the like materials.

The *B. coagulans* isolated active agent can be applied to the solid surface using any of a variety of known methods including, for example, applying a powder, spray drying the probiotic onto the material or soaking the material in a solution containing the probiotic and then using the wetted material or drying the material before use. Porous material may contain the *Bacillus* and/or the isolated active agent in the pores or interstices of the solid material. The *Bacillus* and/or the isolated active agent can be attached by adhesion, such as by attachment to an adhesive layer that is then applied to the skin (e.g., in a bandage or dermal patch). The *Bacillus* and/or the isolated active agent can be impregnated into the solid material during the manufacturing process of the flexible article (e.g., added to a synthetic composition before or during the polymerization process). The pressure and heat resistance of *Bacillus* spores makes them particularly suitable for incorporation into the material during manufacturing. Any of the solid materials carrying *Bacillus* and/or the isolated active agent can be packaged individually or in groups, suitable for holding the treated material using standard packaging materials (e.g., in a shrink wrapper, sealed packet, protective wrapper or dispensing container suitable for holding dry or wet materials).

The article of manufacture can have applied thereon any of the additional/optional components of a therapeutic composition of this invention, including carriers, salts, FOS, fragrances, and the like.

Any of a variety of methods for placing the therapeutic composition onto a subject article can be used, and therefor the invention need not be so limited. However, preferred methods include a "spray-dry" method in which the material is exposed in a low humidity chamber to an atomized mix containing a liquid composition, where the chamber is subsequently exposed to about 80-110 degrees Fahrenheit to dry the liquid, thereby impregnating the material of the article with the components of the composition. A typical load is from 10⁵ to 10⁹ cfu of bacteria/spores per ml of atomizing mix, to place that same amount on about one square inch of external surface of fibrous carrier/article material. The dry article is then ready for storage in a sterile package for use.

### Examples

The following examples relating to this invention are illustrative and should not, of course, be construed as specifically limiting the invention. Moreover, such variations of the invention, now known or later developed, which would be within the purview of one skilled in the art are to be considered to fall within the scope of the present invention hereinafter claimed.

### Example 1: Antimicrobial Activity of B. coagulans

The ability of *B. coagulans* to inhibit various fungal pathogens was demonstrated using an *in vitro* assay. The tested fungal strains of *Trichophyton* species are available from the American Type Culture Collection (ATCC) (Rockville, Maryland) and their ATCC accession numbers are shown in Table 2. In the assay, potato-dextrose plates (DIFCO®, Detroit, MI) were prepared using standard procedures and were inoculated individually with a confluent bed (about 1.7 x 10⁶) of various species of the fungus *Trichophyton*. Inhibition by *B. coagulans* was tested by placing on the plate about 1.5 x 10⁶ colony forming units (CFU) in 10 µl of broth or buffer, plated directly in the center of the potato-dextrose plate with one test locus per plate. The size of each test locus was about 8 mm in diameter and a minimum of three tests were performed for each inhibition assay. The negative control was a 10 µl drop of sterile saline solution, and the positive control was a similar volume of 2% miconazole (1-[2-(2,4-dichlorophenyl)-2-[(2,4-dichlorophenyl)methoxy]ethyl]-1H-imidazole in an inert cream. The plates were then incubated for about 18 hr at 30°C when the zone of inhibition was measured. As used herein, "excellent inhibition" means the zone was 10 mm or greater in diameter; and "good inhibition" means the zone was greater than 2 mm in diameter but less than 10 mm in diameter.

The results of *in vitro* inhibition by *B. coagulans* are shown in Table 2. For each of the *Trichophyton* species tested, the disease condition associated with an infection is indicated in column 2 of Table 2. For comparison, no zone of inhibition was seen with the negative control. Good inhibition (about 8.5 mm diameter, mean average of three tests) was seen with the positive control.

**TABLE 2**

| Pathogen | Related Disease | Inhibition Results |
|---|---|---|
| *T. mentagrophytes* (ATCC# 4808) | Tinea pedis (Athlete's Foot) | Excellent |
| | | |
| *T. interdigitale* (ATCC# 9129) | Tinea pedis (Athlete's Foot) | Excellent |
| *T. mentagrophytes* (ATCC# 36107) | Tinea versicolor (Ring Worm) | Excellent |
| | | |
| *T. mentagrophytes* (ATCC# 8125) | Tinea barbae (Face & Neck Inflammation) | Good |
| | | |
| *T. mentagrophytes* (ATCC# 9533) | Tinea pedis | Excellent |
| | | |
| *T. mentagrophytes* (ATCC# 28187) | Tinea pedis | Excellent |
| | | |
| *T. rubrum* (ATCC# 18753) | Mild Dermatophytosis | Good |
| | | |
| *T. yaoundei* (ATCC# 13947) | Ring Worm, Scalp | Good |

Similarly, the ability of *B. coagulans* to inhibit various yeast pathogens was demonstrated *in vitro* for four species of *Candida*, all of which are available from the American Type Culture Collection (Rockville, Maryland) with their ATCC accession numbers shown in Table 3. In the assay, potato-dextrose plates (DIFCO®, Detroit, MI) were prepared using standard procedures and were inoculated individually with a confluent bed about 1.7 × 10⁶ of the four species of *Candida*. Inhibition by *B. coagulans* was tested by placing on the plate about 1.5 x 10⁶ CFU in 10 µl of broth or buffer, plated directly in the center of the potato-dextrose plate with one test locus of about 8 mm in diameter per plate. A minimum of three tests were performed for each inhibition assay. The negative control was a 10 µl drop of a sterile saline solution and the positive control was a 10 µl volume of miconazole cream. The plates were then incubated for about 18 hr at 30°C when the zone of inhibition was measured using the same criteria as defined earlier herein. No inhibition was seen with the negative control and good inhibition (about 8.7 mm diameter, average of three tests) was seen with the positive control.

The results of the *in vitro* tests are shown in Table 3 with the pathological conditions in humans associated with infection by the *Candida* species shown in column 2.

**TABLE 3**

| Species | Pathology | Inhibition Results |
|---|---|---|
| *Candida albicans* (ATCC# 26555) | Chronic Mucocutaneous, Candidiasis, Myositis and Thymoma | Excellent |
| | | |
| *C. albicans* (ATCC# 44203) | Systemic Candidiasis | |
| | | |
| Excellent | *C. albicans* (ATCC# 44807) | |
| | | |
| Yeast and Mycelial Phase | Excellent | *C. tropicalis* (ATCC# 62377) |
| | | |
| Cervical Yeast Infection | Excellent | |

Similarly, the ability of *B. coagulans* to inhibit opportunistic bacterial pathogens was demonstrated *in vitro* for *Pseudomonas aeruginosa* and *Staphylococcus aureus* which are part of a standard bacterial pathogen screen (U.S. Food and Drug Administration) and are commercially available on solid support disks (DIFCO® BACTROL® disk set). In the assay, potato-dextrose plates (DIFCO®) were prepared using standard procedures and were inoculated individually with a confluent bed 1.5 × 10⁶ of each of the four species of bacteria. Inhibition by *B. coagulans* was tested by placing on the plate about 1.5 × 10⁶ CFU in 10 µl of broth or buffer, plated directly in the center of the potato-dextrose plate with one test locus of about 8 mm in diameter per plate. A minimum of three test loci were used for each assay. The negative control was a 10 µl drop of a sterile saline solution and the positive control was a 10 µl volume of glutaraldehyde. The plates were then incubated for about 18 hr at 30°C when the zone of inhibition was measured using the same criteria as defined earlier herein. No inhibition was seen with the negative control and excellent inhibition (about 16.2 mm diameter, average of three tests) was seen with the positive control. Excellent inhibition was also seen for both opportunistic pathogens, *P. aeruginosa* and *S. aureus*.

### Example 2: Formulation of a Therapeutic Composition

| Formulation 1 : Bathing Formulation (per bath/dosage) | |
|---|---|
| *B. coagulans* | 250,000,000 spores (~18 mg) |
| bath salts (sea & mineral salts) | 10 gm |
| fructooligosaccharides (FOS) 1 gm | |
| micro-crystalline cellulose (MCC) | 5 gm |
| fragrance | Trace |

| Forumulation 2 : Topical Ointment (per ml) | |
|---|---|
| *B. coagulans* extract (Example 3B) | 100 ul |
| lanolin | 780 ul |
| Emu oil | 100 ul |
| geranium essential oil | 20 ul |
| fragrance | trace |

| Formulation 3 : Topical Liquid for dropper application (per ml) | |
|---|---|
| *B. coagulans* extract (Eample 3B) | 500 ul |
| Emu oil | 450 ul |
| geranium essential oil | 20 ul |
| Tween-80 detergent | 30 ul |
| fragrance | trace |

| Formulation 4 : Powder (per gram) | |
|---|---|
| *B. coagulans* | 100,000,000 spores (-8 mg) |
| talc | 992 mg |
| powdered lavender fragrance | trace |

### Example 3A: Preparation of B. coagulans Spores

A culture of dried *B. coagulans* spores was prepared as follows. Ten million spores were innoculated into a one liter culture containing 24 gms potato dextrose broth, 10 gms of enzymic digest of poultry and fish tissue, 5 gms of FOS and 10 gms MnSO4. The culture was maintained for 72 hours under a high oxygen environment at 37 degrees Centigrade to produce culture having about 150 billion cells per gram of culture. Thereafter, the culture was filtered to remove culture medium liquid, and the bacterial pellet was resuspended in water and freeze-dried. The freeze-dried powder is then ground to a fine powder using standard good manufacturing practice (GMP). The powder is then combined into Formulation 1 or Formulation 4 as described in Example 2 to form dry powder compositions.

### Example 3B: Preparation of B. coagulans Extracellular Products

A one liter culture of *B. coagulans* was prepared as described in Example 3A except the FOS was ommitted. The culture was maintained for 5 days as described, at which time FOS was added at 5 gm/liter, and the culture was continued. 20 ml of carrot pulp was then added at day 7, and the culture was harvested when the culture became saturated (no substantial cell division). The culture was first autoclaved for 30 minutes at 250 degrees Farenheight, and then centrifuged at 4000 rpm for 15 min. The resulting supernatant was collected and filtered in a Buchner funnel through a 0.8 micron (u) filter, and the filtrate (pass through) was collected and further filtered through a 0.2 u Nalge vacuum filter. The resulting pass-through was collected (about 900 milliliters) to form a liquid containing an extracellular product, and used in inhibition studies.

Following the assay described in Example 1 using *Candida albicans*, one milliliter of the above-produced extracellular product was added to the test plate in place of the bacterium. After the same culturing time, a zone of inhibition of about 10 to 25 milimeters was observed, indicating a potent antimicrobial activity of "excellent" quality, using the terminology of Example 1.

The liquid containing the extracellular product was formulated into a liquid ointment composition for use in direct application onto a tissue using a dropper, such as would be convenient to treat a fungal infection of the toe nail. This liquid ointment was prepared by combining the liquid extracellular product produced above with Emu essential oil in a ratio of about 8:2, and trace fragrances were added to produce an aesthetic component.

Alternatively, one can use any liposomal or oil based transdermal delivery component in place of the Emu oil. The typical ratio of probiotic extracellular product to carrier or delivery component is a range of from about 1 to 90% probiotic, and preferably about 10 to 75% probiotic.

### Example 4: Topical Application to Prevent Diaper Rash

A powder, aerosol spray liquid, or aerosol spray powder containing *B. coagulans* active agent, preferably *B. coagulans* spores, is applied to diapers by the consumer before use. Alternatively, disposable diapers supplied from the manufacturer may contain *B. coagulans* active agent, preferably *B. coagulans* spores, impregnated into the diaper material where it would be adjacent to the child's skin when in use. When the diaper becomes wetted by urine and/or fecal material, the spores are activated, usually within about twenty minutes. *B. coagulans* spore germination and *B. coagulans* growth after spore germination produce sufficient antifungal, including anti-yeast, activity to inhibit growth of yeast and fungal organisms in the diapers and on the child's skin, thus preventing diaper rash or other diaper-associated opportunistic infections.

Alternatively or in addition to treating diapers with *B. coagulans*, the child's skin in the diaper area can be treated with a saturated soft cloth wipe, powder, aerosol spray liquid, aerosol spray powder, lotion, cream or ointment containing *B. cacrgulans* active agent. Optimally, the *B. coagulans* formulation is applied to the child's skin after bathing and/or when the diapers are changed.

Suitable formulations include a powder of talc and optionally fragrance containing about 10⁵ to about 10¹⁰ *B. coagulans* spores per gram. Other suitable powder formulations contains talc, mineral oil, magnesium carbonate, DMDM hydantoin and about 10⁵ to about 10¹⁰ *B. coagulans* spores per gm or about 10⁵ to about 10¹⁰ *B. coagulans* spores per gm of a corn starch and calcium carbonate powder. An aerosol powder that includes an isobutane or other well known propellant made using standard methods is also suitable. An aerosol spray may be formulated by combining about 10⁶ to about 10¹¹ *B. coagulans* spores per gm in isopropyl myristate, about 60% (w/w) SD alcohol 40-B, and isobutane as the propellant using standard methods. A manual pump spray containing about 10⁵ to about 10¹¹ *B. coagulans* spores per gm of a neutral aqueous solution with no chemical propellant is also suitable. A suitable spray formulation includes alcohol, glycerin, purified water and methylparaben in addition to the *B. coagulans* probiotic. A cream formulation includes aloe vera gel, isopropyl myristate, methylparaben, polysorbate 60, propylparaben, purified water, sorbitan monostearate, sorbitol solution, stearic acid and about 10⁵ to about 10¹⁰ *B. coagulans* spores per gm. Another protective cream contains vitamins A and D equivalent to the concentration found in cod liver oil, cetylpalmitate, cotton seed oil, glycerin, glycerol monostearate, optional fragrance, methylparaben, mineral oil, potassium stearate, propylparaben and about 10⁵ to about 10¹⁰ *B. coagulans* spores per gm. An ointment contains cod liver oil, lanolin oil, methylparaben, propylparaben, talc, optional fragrance and about 10⁵ to about 10¹⁰ *B. coagulans* spores per gm. Another ointment formulation includes petrolatum, water, paraffin, propylene glycol, milk protein, cod liver oil, aloe vera gel, optional fragrance, potassium hydroxide, methyl paraben, propyl paraben, vitamins A, D and E and about 10⁵ to about 10¹⁰ *B. coagulans* spores per gm. A soft cloth pad (i.e., a baby wipe) is soaked in an aqueous solution (e.g., water, amphoteric 2, aloe vera gel, DMDM hydantoin or an aqueous solution of 30%-70% alcohol) and about 10⁴ to about 10⁹ *B. coagulans* spores per gm.

### Example 5: Topical Treatment of Vaginal Yeast Infection

Bath products, including granulated or powdered bubble bath, bath crystals, bath salts, bath oils, powders, aerosol microparticulates and the like, for treatment of vaginal *Candida albicans* and/or *C. tropicalis* infections are produced in any of a variety of well-known formulations containing *B. coagulans* spores as follows. For bubble baths, bath crystals, bath salts, bath oils and the like which are placed in bath water, about 5 x 10⁹ *B. coagulans* spores are used per standard bath (about 30 to 100 gal), such that a bath powder composition comprises about 150-200 x 10⁶ spores per gram of powder. For chaffing (talc-type) powders, about 1 x 10⁹ *B. coagulans* spores per gm of talc, powdered oatmeal, cornstarch or similar powdered substance are used. For aerosols of microparticulates, about 1 x 10⁹ *B. coagulans* spores per ml of carrier are used.

A bath oil contains about 10⁹ *B. coagulans* spores per ml of an oil based formulation such as mineral oil, laureth-4, quaternium-18 hectorite and phenylcarbinol. Natural oil based formulations containing about 10⁹ *B. coagulans* spores per ml of a mixture that includes, for example, olive oil, grape seed oil, emu oil, sweet almond oil, geranium oil, grapefruit oil, mandarin oil and peppermint oil are also suitable, with or without fragrance.

A suitable nonsoap emollient cleanser includes sodium octoxynol-2 ethane sulfonate solution in water, petrolatum, octoxynol-3, mineral oil or lanolin oil, cocamide MEA, optional fragrance, imidazolidinyl urea, sodium benzoate, tetrasodium EDTA, methylcellulose, adjusted to pH 6.5 to 7.5, and about 10⁷ to about 10¹⁰ *B. coagulans* spores per gm. Other suitable cleansers include well known water, glycerin and sodium oleate based formulas, adjusted to about pH 7.0, and containing about 10⁷ to about 10¹⁰ *B. coagulans* spores per gm. Hard milled soaps made by standard methods may also include about 10⁷ to about 10¹⁰ *B. coagulans* spores/g because the spores can withstand pressure and heat during soap manufacturing.

A soft cloth towelette soaked in a solution of water, potassium sorbate, disodium EDTA and containing about 10⁶ to about 10⁹ *B. coagulans* spores per towelette can be used to clean the external vaginal area. Additional components to the formulation may include DMDM hydantoin, isopropyl myristate, methylparaben, polysorbate 60, propylene glycol, propylparaben or sorbitan stearate. The disposable towelette is used to gently wipe the perivaginal area and then discarded.

In addition, vaginal suppositories or inserts containing about 1 × 10⁸ *B. coagulans* spores in an inert solid formulation are useful for mucosal treatment of *C. albicans* and/or *C. tropicalis* infections. Such formulations are well known in the art and can be made, for example, from a combination of corn starch, lactose, a metal stearate (e.g., magnesium stearate) and povidone. One to three inserts should be used per day while symptoms (e.g., vaginal itch and/or whitish discharge) are detected, with optimally about one insert per day used for a total of three to seven days, preferably at bedtime.

### Example 6: Prevention and/or Treatment of Opportunistic Skin Infections

Opportunistic skin infections with *Pseudomonas* and or *Staphylococcus* species (typically *P. aeruginosa*, *S. epidermidis*, *S. aureus)* commonly occur in conjunction with skin allergies (e.g., allergic reactions to plant irritants such as poison ivy), bed sores, diabetic lesions or other types of skin lesions. Probiotic formulations containing *B. coagulans* spores (10⁵ to 10¹⁰/ml depending on the formulation and the application) and/or supernatant or filtrate containing extracellular bacteriocins produced by *B. coagulans* are useful for preventing or treating opportunistic skin pathogens. Additionally, probiotic *B. coagulans* formulations are useful to prevent infection with methicillin-resistant *Staphylococcus aureus* (MRSA), particularly following injury or surgical incisions. A water-in-oil or oil-in-water emulsion, cream, lotion, powder, aerosol powder, or aerosol spray containing about 1 × 10⁶ to about 1 × 10⁹ *B. coagulans* spores per ml is used. Some suitable carriers are described herein, and others are well known in the art.

The skin is cleaned with soap and water and dried thoroughly. Then the *B*. *coagulans* containing formulation is applied to the skin, making sure that the formulation reaches between toes, under breasts, under arms, or other areas where the skin may become moist or exhibit friction chafing.

In addition to treating the skin topically with an emulsion, cream, lotion, powder, aerosol powder, or aerosol spray containing *B. coagulans* probiotic, the skin may be cleansed with a probiotic formulation such as described herein.

### Example 7: Treatment of Tineal Fungal Infections

Ringworm (tinea versicolor) is caused by localized infections of the skin of the trunk and neck by dermatophyte fungus which colonizes the outer layer of the skin resulting in generally circular patches of white, brown or pink flaking skin that are often itchy. Once ringworm is detected, the affected area and a surrounding about 1 to 10 cm² area is treated twice daily with a cream or lotion containing 10% by weight *B. coagulans* spores. Suitable carriers are described herein, optimally containing about 10⁵ to about 10¹⁰ *B. coagulans* spores per ml of carrier.

For treatment of tinea cruris (jock itch), a powder containing about 10⁷ to about 10⁹ *B. corrgulans* spores per ml of colloidal silicon dioxide, isopropyl myristate, talc and optional fragrance is applied to the groin area to provide relief of itching, chafing, burning rash and irritation. Treatment is twice daily, generally after bathing and at bedtime, until symptoms are no longer detected.

Clothing, particularly underclothes and nightclothes that come in contact with the trunk and neck are sprayed with an aerosol containing about 1% to about 20% *B. cargulans* active agent in a suitable carrier such as described herein to prevent the spread of the infection to additional areas of the body.

### Example 8: Treatment of Superficial Skin Infections

Superficial infections with *Staphylococcus* species (e.g., *S. aureus*, *S. epidermidis*) of a blocked sweat or sebaceous gland cause pustules, boils, abscesses, styes or carbuncles. These superficial skin infections may be accompanied by a blistering rash, particularly in babies, due to bacterial toxins released by the *Staphylococcus* species.

A water-in-oil or oil-in-water emulsion, cream, lotion, or gel, containing about 1 × 10⁶ to about 1 × 10⁹ *B. coagulans* spores per ml may be used. An exemplary topical gel is prepared by mixing together equal volumes of propylene glycol and water, 1 % by weight hydroxypropyl cellulose (MW 100,000 to 1,000,000) and lyophilized *B. coagulans* culture to a final concentration of 1 x 10⁶ to about 1 x 10⁹ *B. coagulans* spores per ml of the combination, and allowing the stirred mixture to sit for 3 to 5 days to form a gel. Other formulations are presented herein.

The *B. coagulans*-containing emulsion, cream, lotion, or gel is applied to the area of the skin showing superficial skin infections (pustules, boils, abscesses, styes or carbuncles) or rash and gently rubbed into the skin and allowed to air-dry. Applications are at least once per day, preferably two to three times per day (e.g., morning and night), or after each washing of the infected area for those areas which are washed frequently (e.g., the hands or diaper area). Applications are continued until skin inflammation has subsided and the skin appears normal to the observer. In cases where scabbing has occurred in the infected area, once daily applications are continued until the scabs are no longer present.

### Example 9: Acne Treatment

For treatment or prevention of acne vulgaris, a cleanser containing *B. cargulans* active ingredient obtained from a supernatant of bacterial culture is applied daily as a skin care product for removing excess dirt and oil and for preventing opportunistic infection of the skin. A suitable cleanser includes bentonite, cocoamphodipropionate, optional fragrance, glycerin, iron oxides, magnesium silicate, sodium borohydride, sodium chloride, sodium cocoate, sodium tallowate, talc, tetrasodium EDTA, titanium dioxide, trisodium HEDTA, water and about 1% to about 20% (v/v) of an aqueous supernatant or filtrate of a *B. coagulans* culture grown to saturation.

A similar cleanser, particularly for sensitive skin, includes about 30-50% colloidal oatmeal, suspended in a base of water, glycerin, distearyldimonium chloride, petrolatum, isopropyl palmitate, cetyl alcohol, dimethicone, sodium chloride, adjusted to pH about 7.0, and containing about 5% to about 50% (v/v) of an aqueous supernatant or filtrate of a *B. coagulans* culture grown to saturation.

Alternatively, the skin may be cleansed using any well known cleanser and then a cream containing *B. coagulans* active ingredient from a culture supernatant or filtrate is applied to the skin in a thin film about once every two days to about three times daily as needed. A suitable cream includes about 10-12% alcohol (w/w) bentonite, optional fragrance, iron oxides, potassium hydroxide, propylene glycol, titanium dioxide, purified water and about 0.5% to about 60% (v/v) of an aqueous supernatant or filtrate of a *B. coagulans* culture grown to saturation.

The above formulation is suited for treating acne caused by *Propioni bacterium acne* and by *Staphylococcus epidermidis*.

### Example 10: Treatment of Cold Sores or Genital Herpes Lesions

Cold sores, generally around or in the mouth are caused by the virus Herpes simplex I. Similar lesions around the genitals are caused by Herpes simplex II. Herpes simplex infections can also cause painful finger or toe swelling (Whitlow). Both types of Herpes simplex lesions or Whitlow can be treated with a cream, lotion or gel ointment containing about 1 × 10⁷ to about 1 × 10¹⁰ *B. coagulans* spores per ml.

For oral cold sores, a soothing emollient lip balm contains allantoin, petrolatum, titanium dioxide at cosmetically acceptable levels, and about 10⁷ to about 10¹⁰ *B. coagulans* spores per ml. The lip balm may further include a sunscreen (e.g., padimate O). An alternative emollient lip balm contains the same base ingredients mixed to form an emulsion with 0.5% to 20% (v/v) of an aqueous supernatant or filtrate of a *B. coagulans* culture grown to saturation. The lip balm is applied to the lips and affected area to form a light film as a prophylactic when prodromal symptoms are felt (tingling, itching, burning) or when a lesion is visible. The lip balm should be applied as often as needed (e.g., every hour when a lesion is present) and generally once per day at bedtime.

For oral cold sores, the *B. coagulans* spores or extracellular agent in culture supernatant or filtrate may be formulated into a semisolid lip balm containing about 20-40% white petrolatum, wax paraffin, mineral oil, isopropyl lanolate, camphor, lanolin, isopropyl myristate, cetyl alcohol, carnuba wax, methylparaben, propylparaben, titanium dioxide and optionally fragrance and coloring agents.

For genital herpes lesions, a cream or ointment is formulated using standard methods as described herein containing about 1 × 10⁷ to about 1 × 10¹⁰ *B. coagulans* spores per ml and/or 0.5% to 20% (v/v) of an aqueous supernatant or filtrate of a *B. coagulans* culture grown to saturation. The cream or ointment is applied at least twice daily as needed.

### Example 11: Ear Drops or Ear Wash Containing B. coagulans Spores

For prevention or treatment of outer ear canal infections, an aqueous formulation that includes about 1 × 10⁵ to about 1 × 10⁸ *B. coagulans* spores per ml and/or 0.1% to 15% (v/v) of an aqueous supernatant or filtrate of a *B. coagulans* culture grown to saturation is used. The spores and/or supernatant is added to a sterile aqueous solution of 5-50% glycerin, 0.1-5% propylene glycol and sodium stannate or sodium chloride. An alternative formulation includes about 1 × 10⁵ to about 1 × 10⁸ *B. coagulans* spores per ml and/or 0.1% to 15% (v/v) of an aqueous supernatant or filtrate of a *B. coagulans* culture grown to saturation in a sterile aqueous solution of 0.5-25% glycerin, 5-10% alcohol and polysorbate 20

To apply, the user tilts the head sideways and about 3 to 10 drops of the ear formulation is added to the ear using a standard dropper applicator, without having the applicator enter the ear canal. The head is kept tilted for several minutes or the ear is lightly plugged with a wad of cotton to allow the solution to remain in the ear for up to 15 minutes. Then the head is tilted and excess solution is allowed to drain from the ear. Gentle washing with a soft rubber bulb ear syringe containing warm water may be used to remove excess. The probiotic solution can be applied occasionally or daily for up to about five days. A physician should be consulted if there is drainage, discharge, rash, severe irritation in the ear or if the patient experiences dizziness.

### Example 12: Prophylactic or Treatment for Athlete's Foot

For prevention or treatment of athlete's foot (tineal fungal infection), the feet are washed with soap and water, dried thoroughly and a powder, cream, lotion, ointment or gel, such as those described in the above examples is applied to the entire foot area. Optimally, the formulation includes about 10⁵ to about 10⁸ *B. coagulans* spores or 0.5% to 20% *B. coagulans* supernatant or filtrate. Daily treatments are continued as needed.

Additionally, athlete's foot may be prevented or treated by using a standard insole insert (e.g. a fabric, fiber or synthetic foam) having sprayed on the surface or impregnated therein with the *B. coagulans* probiotic or extracellular antifungal product. Such treated insoles may be worn daily for up to two to three months when they are replace with fresh treated insoles.

## Claims

1. Use of a composition comprising a *Bacillus coagulans* bacterium or extracellular product thereof in a pharmaceutically acceptable carrier for the manufacture of a medicament for preventing bacterial, yeast, fungal or viral infection, wherein the medicament is suitable for topical application to skin or a mucous membrane of a mammal, and wherein the carrier is in the form of an emulsion, cream, lotion, gel, oil, ointment, suspension, aerosol spray, powder, aerosol powder or semi-solid formulation.

2. The use of Claim 1, wherein the *Bacillus coagulans* bacterium is included in the composition in the form of spores.

3. The use of Claim 1 wherein said composition comprises 10³ to 10¹² viable bacterium or spore per gram of composition.

4. The use of Claim 1 wherein said medicament is for administration of from 10⁸ to 10¹⁰ viable bacterium or spore per day.

5. The use of Claim 1 wherein said medicament is for administration of from 5 x 10⁸ to 10⁹ viable bacterium or spore per day.

6. The use of Claim 1 wherein the composition comprises an effective amount of a fructo-oligosccharide (FOS).

7. The use of Claim 6 wherein said FOS is present in an amount of from about 10 to 1000 milligrams per gram of composition.

8. The method of Claim 7 wherein said FOS is present in an amount of from about 100 to 500 milligrams per gram of composition.

9. The use of Claim 1, wherein the medicament inhibits growth of one or more microbes selected from the group consisting of *Staphylococcus* species, *Pseudomonas* species, *Escherichia coli, Proteus* species, *Klebsiella* species, *Candida* species and *Trichophyton* species.

10. Use of a composition comprising an extracellular product of a *Bacillus coagulans* strain in a pharmaceutically acceptable carrier for the manufacture of a medicament for inhibiting growth of bacteria, yeast, fungus, virus or a combination thereof, wherein the medicament is suitable for topical application to skin or a mucous membrane of a mammal, and wherein the carrier is in the form of an emulsion, cream, lotion, gel, oil, ointment, suspension, aerosol spray, powder, aerosol powder or semi-solid formulation.

11. The use of Claim 10 wherein said composition further comprises emu oil.

12. Use of a composition comprising a *Bacillus coagulans* bacterium or extracellular product thereof applied to a solid surface for the manufacture of a medicament for inhibiting growth of bacteria, yeast, fungus, virus or a combination thereof wherein the medicament is suitable for application to skin or mucous membrane of a mammal.

13. The use of Claim 12, wherein the solid surface comprises a flexible article selected from the group consisting of a diaper, pliable material for wiping skin or a mucous membrane, dermal patch, adhesive tape, absorbent pad, tampon or article of clothing.

14. The use of Claim 12, wherein the application to a solid surface comprises impregnating the composition into a fibrous or nonfibrous solid matrix.

15. The use of Claim 12, wherein the *Bacillus* species is included in the composition in the form of spores.

16. The use of Claim 12, wherein the *Bacillus* species is included in the composition in the form if a dried cell mass.

17. The use of Claim 12 wherein said composition comprises contains 10³ to 10¹² viable bacterium or spore per gram of composition.

18. The use of Claim 12 wherein the composition comprises an effective amount of a fructo-oligosccharide (FOS).

19. The use of Claim 18 wherein said FOS is present in an amount of from about 10 to 1000 milligrams per gram of composition.

20. The use of Claim 18 wherein said FOS is present in an amount of from about 100 to 500 milligrams per gram of composition.

21. A composition comprising a *Bacillus coagulans* bacterium or extracellular product thereof in a pharmaceutically acceptable carrier suitable for topical application to skin or a mucous membrane of a mammal, wherein the carrier is in the form of an emulsion, cream, lotion, gel, oil, ointment, suspension, aerosol spray, powder, aerosol powder or semi-solid formulation.

22. The composition of Claim 21, wherein the *Bacillus coagulans* bacterium is included in the composition in the form of spores.

23. The composition of Claim 21, wherein the *Bacillus coagulans* bacterium is included in the composition in the form of a dried cell mass.

24. The composition of Claim 21 wherein said composition comprises contains 10³ to 10¹² viable bacterium or spore per gram of composition.

25. The composition of Claim 21 further comprising an effective amount of an fructo-oligosccharide (FOS).

26. The composition of Claim 25 wherein said FOS is present in an amount of from about 10 to 1000 milligrams per gram of composition.

27. The composition of Claim 25 wherein said FOS is present in an amount of from about 100 to 500 milligrams per gram of composition.

28. The composition of Claim 21, wherein the extracellular product is a supernatant or filtrate of a culture of a *Bacillus coagulans* strain.

29. The composition of Claim 21 further comprising emu oil.

30. The composition of Claim 21, further comprising an antifungal agent.

31. The composition of Claim 21, further comprising an antimicrobial agent.

32. The composition of Claim 21, further comprising an antiviral agent.

33. The composition of Claim 30, wherein said antifungal agent is miconozole.

34. The composition of Claim 21, wherein said composition further comprises white petrolatum, isopropyl myristate, lanolin, lanolin alcohol, mineral oil, fragrant oil, essential oil, nasturtium extract oil, sorbitan mono-oleate, propylene glycol, cetylstearyl alcohol, hydroxypropyl cellulose, polyoxyl stearate, or sodium lauryl sulfate.

35. The composition of Claim 21, further comprising an antioxidant, a buffering agent, a sunscreen, or a cosmetic agent.

36. A flexible article which can be worn on or wiped on the skin or mucous membrane, said article including a composition of *Bacillus coagulans* or *Bacillus coagulans* active agent.

37. A flexible article as claimed in claim 36, which is selected from the group consisting of diapers, bandaids, towelettes such as baby wipes or feminine hygiene towelettes, tampons, dermal patches, adhesive tape, absorbent pads, articles of clothing such as underclothes or sleeping apparel, bath towels and wash cloths.

38. A flexible article as claimed in claim 36 or claim 37, which is made from fibrous woven or knitted material, non-woven material such as felt and batting, fibre balls of cotton, rayon, cellulose, occlusive or non-occlusive films or membranes, synthetic polymer fibres, films, membranes, or foams such as nylon, polytetrafluoroethylene, polystyrene, polycarbonate, polyvinylchloride and polysulphone foams.

39. A flexible article as claimed in any of claims 36-38, wherein said *Bacillus coagulans* comprises vegetative bacteria; spores, a dried cell mass or an extracellular product of a *Bacillus coagulans* species.

40. A flexible article as claimed in any of claims 36-39, wherein said *Bacillus coagulans* comprises cells or spores which have been lyophilised, spray dried, air dried and/or frozen.

41. A flexible article as claimed in claim 39, wherein said extracellular product is a supernatant or a filtrate of a culture of an isolated *Bacillus coagulans* species.

42. A flexible article as claimed in any of claims 36-41, wherein said *Bacillus coagulans* is *Bacillus coagulans* Hammer.

43. A flexible article as claimed in any of claims 36-42, wherein said composition is formulated as an emulsion, cream, lotion, gel, oil, ointment, suspension, aerosol spray, powder, aerosol powder or semi-solid formulation.

44. A flexible article as claimed in any of claims 36-43, which comprises from 10⁵ to 10⁹ cfu of bacteria/spores per square inch of the external surface of said article.

45. A flexible article as claimed in any of claims 36-44, wherein said composition *of Bacillus coagulans* or *Bacillus coagulans* active agent is applied to a surface of said article or is impregnated into said article.

46. A flexible article as claimed in claim 45, wherein said composition of *Bacillus coagulans* or *Bacillus coagulans* active agent is applied as a powder to the article, or is spray-dried onto the article or soaked into the article in the form of a solution.

47. A flexible article as claimed in any of claims 36-46, wherein said article comprises a porous material and said *Bacillus* or active agent is contained in the pores or interstices of said material.

48. A flexible article as claimed in any of claims 36-47, further comprising a quantity of a fructooligosaccharide.

49. A flexible article as claimed in claim 48, wherein said fructooligosaccharide has a polymer chain length between about 4 and 200 sugar units.

50. A flexible article as claimed in any of claims 36-49, which is disposable.

51. A flexible article as claimed in any of claims 36-50, further comprising one or more antimicrobial agents, antiviral agents, and/or antifungal agents.

## Patentansprüche

1. Verwendung einer Zusammensetzung, umfassend ein *Bacillus coagulans*-Bakterium oder ein extrazelluläres Produkt davon, in einem pharmazeutisch annehmbaren Träger für die Herstellung eines Medikamentes zum Verhindern einer bakteriellen, Hefe-, Pilz- oder Vireninfektion, wobei das Medikament für die topische Aufbringung auf die Haut oder eine Schleimhautmembran eines Säugers geeignet ist, und wobei der Träger in der Form einer Emulsion, Creme, Lotion, Gel, Öl, Salbe, Suspension, Aerosol-Spray, Pulver, Aerosol-Pulver oder einer halbfesten Formulierung vorliegt.

2. Verwendung von Anspruch 1, wobei das *Bacillus coagulans*-Bakterium in der Zusammensetzung in der Form von Sporen eingeschlossen ist.

3. Verwendung von Anspruch 1, wobei die Zusammensetzung 10³ bis 10¹² lebensfähige Bakterien oder Sporen pro Gramm der Zusammensetzung umfasst.

4. Verwendung von Anspruch 1, wobei das Medikament für die Verabreichung von 10⁸ bis 10¹⁰ lebensfähigen Bakterien oder Sporen pro Tag ist.

5. Verwendung von Anspruch 1, wobei das Medikament für die Verabreichung von 5 x 10⁸ bis 10⁹ lebensfähigen Bakterien oder Sporen pro Tag ist.

6. Verwendung von Anspruch 1, wobei die Zusammensetzung eine wirksame Menge eines Fructo-Oligosaccharids (FOS) umfasst.

7. Verwendung von Anspruch 6, wobei das FOS in einer Menge von etwa 10 bis 1000 Milligramm pro Gramm der Zusammensetzung vorhanden ist.

8. Verwendung von Anspruch 7, wobei das FOS in einer Menge von etwa 100 bis 500 Milligramm pro Gramm der Zusammensetzung vorhanden ist.

9. Verwendung von Anspruch 1, wobei das Medikament das Wachstum von einer oder mehreren Mikroben inhibiert, die aus der Gruppe gewählt sind, welche aus *Staphylococcus*-Spezies, Pseudomonas-Spezies, *Escherichia coli, Proteus*-Spezies, *Klebsiella*-Spezies, *Candida*-Spezies und *Trichophyton*-Spezies besteht.

10. Verwendung einer Zusammensetzung, umfassend ein extrazelluläres Produkt eines *Bacillus coagulans*-Stamms in einem pharmazeutisch annehmbaren Träger für die Herstellung eines Medikamentes zum Inhibieren des Wachstums von Bakterien, Hefe, Pilzen, Viren oder einer Kombination davon, wobei das Medikament für die topische Aufbringung auf die Haut oder eine Schleimhautmembran eines Säugers geeignet ist, und wobei der Träger in der Form einer Emulsion, Creme, Lotion, Gel, Öl, Salbe, Suspension, Aerosol-Spray, Pulver, Aerosol-Pulver oder einer halbfesten Formulierung vorliegt.

11. Verwendung von Anspruch 10, wobei die Zusammensetzung ferner Emu-Öl umfasst.

12. Verwendung einer Zusammensetzung, umfassend ein *Bacillus coagulans*-Bakterium oder ein extrazelluläres Produkt davon, aufgebracht auf eine feste Oberfläche, für die Herstellung eines Medikamentes zum Inhibieren des Wachstums von Bakterien, Hefe, Pilzen, Viren oder einer Kombination davon, wobei das Medikament für die Aufbringung auf die Haut oder eine Schleimhautmembran eines Säugers geeignet ist.

13. Verwendung von Anspruch 12, wobei die feste Oberfläche einen flexiblen Gegenstand umfasst, der aus der Gruppe gewählt ist, welche aus einer Windel, geschmeidigem Material zum Abwischen von Haut oder einer Schleimhautmembran, einem Hautpflaster, Klebeband, einem absorptionsfähigen Polster, einem Tampon oder einem Kleidungsstück besteht.

14. Verwendung von Anspruch 12, wobei die Aufbringung auf eine feste Oberfläche das imprägnieren der Zusammensetzung in eine faserartige oder nichtfaserartige feste Matrix umfasst.

15. Verwendung von Anspruch 12, wobei die Bacillus-Spezies in der Zusammensetzung in der Form von Sporen eingeschlossen ist.

16. Verwendung von Anspruch 12, wobei die *Bacillus*-Spezies in der Zusammensetzung in der Form einer getrockneten Zellmasse eingeschlossen ist.

17. Verwendung von Anspruch 12, wobei die Zusammensetzung 10³ bis 10¹² lebensfähige Bakterien oder Sporen pro Gramm der Zusammensetzung umfasst.

18. Verwendung von Anspruch 12, wobei die Zusammensetzung eine wirksame Menge eines Fructo-Oligosaccharids (FOS) umfasst.

19. Verwendung von Anspruch 18, wobei das FOS in einer Menge von etwa 10 bis 1000 Milligramm pro Gramm der Zusammensetzung vorhanden ist.

20. Verwendung von Anspruch 18, wobei das FOS in einer Menge von etwa 100 bis 500 Milligramm pro Gramm der Zusammensetzung vorhanden ist.

21. Zusammensetzung, umfassend ein *Bacillus* coagulans-Bakterium oder extrazelluläres Produkt davon in einem pharmazeutisch annehmbaren Träger, der für topische Aufbringung auf die Haut oder eine Schleimhautmembran eines Säugers geeignet ist, wobei der Träger in der Form einer Emulsion, Creme, Lotion, Gel, Öl, Salbe, Suspension, Aerosol-Spray, Pulver, Aerosol-Pulver oder einer halbfesten Formulierung vorliegt.

22. Zusammensetzung von Anspruch 21, wobei das *Bacillus coagulans*-Bakterium in der Zusammensetzung in der Form von Sporen eingeschlossen ist.

23. Zusammensetzung von Anspruch 21, wobei das *Bacillus coagulans*-Bakterium in der Zusammensetzung in der Form einer getrockneten Zeilmasse eingeschlossen ist.

24. Zusammensetzung von Anspruch 21, wobei die Zusammensetzung 10³ bis 10¹² lebensfähige Bakterien oder Sporen pro Gramm der Zusammensetzung umfasst.

25. Zusammensetzung von Anspruch 21, ferner umfassend eine wirksame Menge eines Fructo-Otigosaccharids (FOS).

26. Zusammensetzung von Anspruch 25, wobei das FOS in einer Menge von etwa 10 bis 1000 Milligramm pro Gramm der Zusammensetzung vorhanden ist.

27. Zusammensetzung von Anspruch 25, wobei das FOS in einer Menge von etwa 100 bis 500 Milligramm pro Gramm der Zusammensetzung vorhanden ist..

28. Zusammensetzung von Anspruch 21, wobei das extrazelluläre Produkt ein Überstand oder Filtrat einer Kultur eines *Bacillus coagulans*-Stammes ist.

29. Zusammensetzung von Anspruch 21, welche ferner Emu-Öl umfasst.

30. Zusammensetzung von Anspruch 21, welche ferner ein Antipilzmittel umfasst.

31. Zusammensetzung von Anspruch 21, welche ferner ein antimikrobielles Mittel umfasst.

32. Zusammensetzung von Anspruch 21, welche ferner ein antivirales Mittel umfasst.

33. Zusammensetzung von Anspruch 30, wobei das Antipilzmittel Miconozol ist.

34. Zusammensetzung von Anspruch 21, wobei die Zusammensetzung ferner weißes Petrolatum, Isopropylmyristat, Lanolin, Lanolinalkohol, Mineralöl, Duftöl, ätherisches Öl, Nasturtium-Extrakt-Öl, Sorbitanmonooleat, Propylenglykol, Cetylstearylalkohol, Hydroxypropylcellulose, Polyoxylstearat oder Natriumlaurylsulfat umfasst.

35. Zusammensetzung von Anspruch 21, ferner umfassend ein Antioxidationsmittel, ein Puffermittel, ein Sonnenschutzmittel oder ein kosmetisches Mittel.

36. Flexibler Gegenstand, welcher auf der Haut oder Schleimhautmembran getragen oder gewischt werden kann, wobei der Gegenstand eine Zusammensetzung von *Bacillus coagulans* oder Wirkstoff von *Bacillus coagulans* einschließt.

37. Flexibler Gegenstand, wie beansprucht in Anspruch 36, welcher gewählt wird aus der Gruppe, die aus Windeln, Verbänden, Hygienetüchern wie Babywischtüchem oder Damenbinden, Tampons, Hautpflastern, Klebeband, absorptionsfähigen Polstern, Kleidungsstücken, wie Unterwäsche oder Schlafanzügen, Badehandtüchem und Waschtüchern bzw. -lappen, besteht.

38. Flexibler Gegenstand, wie beansprucht in Anspruch 36 oder Anspruch 37, welcher aus faserartigem gewebten oder gestrickten Material, Nonwoven-Material wie Filz und Wickelstoff, Faserbällen aus Baumwolle, Rayon, Cellulose, okklusiven oder nicht-okklusiven Folien oder Membranen, synthetischen Polymerfasem, -folien, -membranen oder Schäumen, wie Nylon-, Polytetrafluorethylen-, Polystyrol-, Polycarbonat-, Polyvinylchlorid- und Polysulfonschäumen, hergestellt ist.

39. Flexibler Gegenstand, wie beansprucht in mindestens einem der Ansprüche 36-38, wobei der *Bacillus coagulans* vegetative Bakterien, Sporen, eine getrocknete Zellmasse oder ein extrazelluläres Produkt einer *Bacillus coagulans*-Spezies umfasst.

40. Flexibler Gegenstand, wie beansprucht in mindestens einem der Ansprüche 36-39, wobei der *Bacillus coagulans* Zellen oder Sporen, welche lyophilisiert, sprühgetrocknet, luftgetrocknet und/oder gefroren worden sind, umfasst.

41. Flexibler Gegenstand, wie beansprucht in Anspruch 39, wobei das extrazelluläre Produkt ein Überstand oder ein Filtrat einer Kultur einer isolierten *Bacillus coagulans*-Spezies ist.

42. Flexibler Gegenstand, wie beansprucht in mindestens einem der Ansprüche 36-41, wobei es sich bei dem *Bacillus coagulans* um *Bacillus coagulans* Hammer handelt.

43. Flexibler Gegenstand, wie beansprucht in mindestens einem der Ansprüche 36-42, wobei die Zusammensetzung als Emulsion, Creme, Lotion, Gel, Öl, Salbe, Suspension, Aerosol-Spray, Pulver, Aerosol-Pulver oder halbfeste Formulierung formuliert ist.

44. Flexibler Gegenstand, wie beansprucht in mindestens einem der Ansprüche 36-43, welcher 10⁵ bis 10⁹ cfu Bakterien/Sporen pro Quadratinch der äußeren Oberfläche des Gegenstands umfasst.

45. Flexibler Gegenstand, wie beansprucht in mindestens einem der Ansprüche 36-44, wobei die Zusammensetzung von *Bacillus coagulans* oder *Bacillus coagulans*-Wirkstoff auf die Oberfläche des Gegenstands aufgebracht wird oder in den Gegenstand imprägniert wird.

46. Flexibler Gegenstand, wie beansprucht in Anspruch 45, wobei die Zusammensetzung von *Bacillus coagulans* oder *Bacillus coagulans*-Wirkstoff als ein Pulver auf den Gegenstand aufgebracht wird oder auf den Gegenstand sprühgetrocknet wird oder in der Form einer Lösung in den Gegenstand einsickern gelassen wird.

47. Flexibler Gegenstand, wie beansprucht in mindestens einem der Ansprüche 36-46, wobei der Gegenstand ein poröses Material umfasst, und der *Bacillus* oder Wirkstoff in den Poren oder Zwischenräumen des Materials enthalten ist.

48. Flexibler Gegenstand, wie beansprucht in mindestens einem der Ansprüche 36-47, ferner umfassend eine Menge an einem Fructooligosaccharid.

49. Flexibler Gegenstand, wie beansprucht in Anspruch 48, wobei das Fructooligosaccharid eine Polymerkettenlänge zwischen etwa 4 und 200 Zuckereinheiten aufweist.

50. Flexibler Gegenstand, wie beansprucht in mindestens einem der Ansprüche 36-49, welcher für einmaligen Gebrauch bzw. Wegwerfen vorgesehen ist.

51. Flexibler Gegenstand, wie beansprucht in mindestens einem der Ansprüche 36-50, ferner umfassend ein oder mehrere antimikrobielle Mittel, antivirale Mittel und/oder Antipilzmittel.

## Revendications

1. Utilisation d'une composition comprenant une bactérie *Bacillus coagulans* ou un produit extracellulaire de celle-ci dans un support pharmaceutiquement acceptable pour la fabrication d'un médicament pour prévenir les infections bactériennes, par levures, fongiques ou virales, dans laquelle le médicament est adapté pour une application topique sur la peau ou une membrane muqueuse d'un mammifère, et dans laquelle le support est sous la forme d'une émulsion, d'une crème, d'une lotion, d'un gel, d'une huile, d'un onguent, d'une suspension, d'un spray aérosol, d'une poudre, d'une poudre aérosol ou d'une formulation semi-solide.

2. Utilisation selon la revendication 1, dans laquelle la bactérie *Bacillus coagulans* est comprise dans la composition sous la forme de spores.

3. Utilisation selon la revendication 1, dans laquelle ladite composition comprend 10³ à 10¹² bactéries ou spores, viables, par gramme de composition.

4. Utilisation selon la revendication 1, dans laquelle ledit médicament est pour une administration de 10⁸ à 10¹⁰ bactéries ou spores, viables, par jour.

5. Utilisation selon la revendication 1, dans laquelle ledit médicament est pour une administration de 5 x 10⁸ à 10⁹ bactéries ou spores, viables, par jour.

6. Utilisation selon la revendication 1, dans laquelle la composition comprend une quantité efficace d'un fructo-oligosaccharide (FOS).

7. Utilisation selon la revendication 6, dans laquelle ledit FOS est présent en une quantité d'environ 10 à 1000 milligrammes par gramme de composition.

8. Procédé selon la revendication 7, dans lequel ledit FOS est présent en une quantité d'environ 100 à 500 milligrammes par gramme de composition.

9. Utilisation selon la revendication 1, dans laquelle le médicament inhibe la croissance d'un ou plusieurs microbes choisis dans le groupe constitué par l'espèce *Staphylococcus,* l'espèce *Pseudomonas, Escherichia coli,* l'espèce *Proteus,* l'espèce *Klebsiella,* l'espèce *Candida* et l'espèce *Trichophyton*.

10. Utilisation d'une composition comprenant un produit extracellulaire d'une souche de *Bacillus coagulans* dans un support pharmaceutiquement acceptable pour la fabrication d'un médicament pour inhiber la croissance de bactéries, levures, champignons, virus ou une combinaison de ceux-ci, dans laquelle le médicament est adapté pour une application topique sur la peau ou une membrane muqueuse d'un mammifère, et dans laquelle le support est sous la forme d'une émulsion, d'une crème, d'une lotion, d'un gel, d'une huile, d'un onguent, d'une suspension, d'un spray aérosol, d'une poudre, d'une poudre aérosol ou d'une formulation semi-solide.

11. Utilisation selon la revendication 10, dans laquelle ladite composition comprend en outre de l'huile d'émeu.

12. Utilisation d'une composition comprenant une bactérie *Bacillus coagulans* ou un produit extracellulaire de celle-ci, appliquée sur une surface solide pour la fabrication d'un médicament pour inhiber la croissance de bactéries, levures, champignons, virus ou une combinaison de ceux-ci, dans laquelle le médicament est adapté pour une application sur la peau ou une membrane muqueuse d'un mammifère.

13. Utilisation selon la revendication 12, dans laquelle la surface solide comprend un objet flexible choisi dans le groupe constitué par une toile, un matériau pliable à passer sur la peau ou une membrane muqueuse, un patch dermique, un ruban adhésif, une compresse absorbante, un tampon ou un article d'habillement.

14. Utilisation selon la revendication 12, dans laquelle l'application sur une surface solide comprend l'imprégnation de la composition dans une matrice solide fibreuse ou non-fibreuse.

15. Utilisation selon la revendication 12, dans laquelle l'espèce *Bacillus* est comprise dans la composition sous la forme de spores.

16. Utilisation selon la revendication 12, dans laquelle l'espèce *Bacillus* est comprise dans la composition sous la forme d'une masse cellulaire sèche.

17. Utilisation selon la revendication 12, dans laquelle ladite composition contient 10³ à 10¹² bactéries ou spores, viables, par gramme de composition.

18. Utilisation selon la revendication 12, dans laquelle la composition comprend une quantité efficace d'un fructo-oligosaccharide (FOS).

19. Utilisation selon la revendication 18, dans laquelle ledit FOS est présent en une quantité d'environ 10 à 1000 milligrammes par gramme de composition.

20. Utilisation selon la revendication 18, dans laquelle ledit FOS est présent en une quantité d'environ 100 à 500 milligrammes par gramme de composition.

21. Composition comprenant une bactérie *Bacillus coagulans* ou un produit extracellulaire de celle-ci, dans un support pharmaceutiquement acceptable adapté pour une application topique sur la peau ou une membrane muqueuse d'un mammifère, dans laquelle le support est sous la forme d'une émulsion, d'une crème, d'une lotion, d'un gel, d'une huile, d'un onguent, d'une suspension, d'un spray aérosol, d'une poudre, d'une poudre aérosol ou d'une formulation semi-solide.

22. Composition selon la revendication 21, dans laquelle la bactérie *Bacillus coagulans* est comprise dans la composition sous la forme de spores.

23. Composition selon la revendication 21, dans laquelle la bactérie *Bacillus coagulans* est comprise dans la composition sous la forme d'une masse cellulaire sèche.

24. Composition selon la revendication 21, dans laquelle ladite composition comprend 10³ à 10¹² bactéries ou spores, viables, par gramme de composition.

25. Composition selon la revendication 21, comprenant en outre une quantité efficace d'un fructo-oligosaccharide (FOS).

26. Composition selon la revendication 25, dans laquelle ledit FOS est présent en une quantité d'environ 10 à 1000 milligrammes par gramme de composition.

27. Composition selon la revendication 25, dans laquelle ledit FOS est présent en une quantité d'environ 100 à 500 milligrammes par gramme de composition.

28. Composition selon la revendication 21, dans laquelle le produit extracellulaire est un surnageant ou un filtrat d'une culture d'une souche de *Bacillus coagulans*.

29. Composition selon la revendication 21, comprenant en outre de l'huile d'émeu.

30. Composition selon la revendication 21, comprenant en outre un agent antifongique.

31. Composition selon la revendication 21, comprenant en outre un agent antimicrobien.

32. Composition selon la revendication 21, comprenant en outre un agent antiviral.

33. composition selon la revendication 30, dans laquelle ledit agent antifongique est le miconazole.

34. Composition selon la revendication 21, dans laquelle ladite composition comprend en outre de la vaseline blanche, du myristate d'isopropyle, de la lanoline, de l'alcool de lanoline, une huile minérale, une huile de fragrance, une huile essentielle, une huile d'extrait de capucine, du monooléate de sorbitane, du propylèneglycol, de l'alcool cétylstéarylique, de l'hydroxypropyl cellulose, du polyoxyl stéarate, ou du laurylsulfate de sodium.

35. Composition selon la revendication 21, comprenant en outre un antioxydant, un agent tampon, un écran solaire, ou un agent cosmétique.

36. Objet flexible qui peut être porté ou passé sur la peau ou la membrane muqueuse, ledit objet comprenant une composition de *Bacillus coagulans* ou un agent actif de *Bacillus coagulans*.

37. Objet flexible selon la revendication 36, qui est choisi dans le groupe constitué par des toiles, des pansements, des lingettes telles que des lingettes pour bébé ou des lingettes pour l'hygiène féminine, des tampons, des patchs dermiques, des rubans adhésifs, des compresses absorbantes, des articles de vêtement tels que les sous-vêtements ou les vêtements de couchage, les serviettes de bain et les gants de toilette.

38. Objet flexible selon la revendication 36 ou la revendication 37, qui est fait d'un matériau fibreux tissé ou tricoté, d'un matériau non-tissé tel que le feutre et la nappe ouatée, de balles de fibres de coton, de rayonne, de cellulose, de films ou membranes occlusifs ou non-occlusifs, de fibres, films, membranes de polymère synthétique, ou de mousses telles que les mousses de nylon, de polytétrafluoroéthylène, de polystyrène, de polycarbonate, de polyvinylchlorure et de polysulfone.

39. Objet flexible selon l'une quelconque des revendications 36 à 38, dans lequel ledit *Bacillus coagulans* comprend des bactéries végétatives, des spores, une masse cellulaire sèche ou un produit extracellulaire d'une espèce de *Bacillus coagulans*.

40. Objet flexible selon l'une quelconque des revendications 36 à 39, dans lequel ledit Bacillus *coagulans* comprend des cellules ou des spores qui ont été lyophilisées, séchées par pulvérisation, séchées à l'air et/ou congelées.

41. Objet flexible selon la revendication 39, dans lequel ledit produit extracellulaire est un surnageant ou un filtrat d'une culture d'une espèce isolée de *Bacillus coagulans*.

42. Objet flexible selon l'une quelconque des revendications 36 à 41, dans lequel ledit *Bacillus coagulans* est *Bacillus coagulans* Hammer.

43. Objet flexible selon l'une quelconque des revendications 36 à 42, dans lequel ladite composition est formulée en émulsion, crème, lotion, gel, huile, onguent, suspension, spray aérosol, poudre, poudre aérosol ou formulation semi-solide.

44. Objet flexible selon l'une quelconque des revendications 36 à 43, qui comprend de 10⁵ à 10⁹ cfu de bactéries / spores par pouce carré de la surface externe dudit objet.

45. Objet flexible selon l'une quelconque des revendications 36 à 44, dans lequel ladite composition de *Bacillus coagulans* ou d'agent actif de *Bacillus coagulans* est appliquée sur la surface dudit objet ou est imprégnée dans ledit objet.

46. Objet flexible selon la revendication 45, dans lequel ladite composition de *Bacillus coagulans* ou d'agent actif de *Bacillus coagulans* est appliquée en tant que poudre sur l'objet, ou est séchée par pulvérisation sur l'objet ou est amenée à pénétrer dans l'objet sous forme de solution.

47. Objet flexible selon l'une quelconque des revendications 36 à 46, lequel objet comprend un matériau poreux et ledit *Bacillus* ou agent actif est contenu dans les pores ou interstices dudit matériau.

48. Objet flexible selon l'une quelconque des revendications 36 à 47, comprenant en outre une certaine quantité de fructo-oligosaccharide.

49. Objet flexible selon la revendication 48, dans lequel ledit fructo-oligosaccharide a une longueur de chaîne polymère d'entre environ 4 et 200 unités de sucre.

50. Objet flexible selon l'une quelconque des revendications 36 à 49, qui est jetable.

51. Objet flexible selon l'une quelconque des revendications 36 à 50, comprenant en outre un ou plusieurs agents antimicrobiens, agents antiviraux, et / ou agents antifongiques.
